# EUROPEAN PATENT SPECIFICATION

(11) **EP 2 152 851 B1**
(45) Date of publication and mention of the grant of the patent: **25.04.2018**
(21) Application number: 08731395.3
(22) Date of filing: 05.03.2008
(51) Int. Cl.: C12M 3/06, C12N 5/00

(54) **IMPROVED BIOREACTOR SURFACES**
VERBESSERTE BIOREAKTOROBERFLÄCHEN
SURFACES DE BIORÉACTEURS AMÉLIORÉES

(30) Priority: 06.04.2007 US 910502 P
(43) Date of publication of application: 17.02.2010
(73) Proprietor: Terumo BCT, Inc., Lakewood, CO 80215 (US)
(72) Inventor: ANTWILER, Glen Delbert, Lakewood, Colorado 80215 (US)
(74) Representative: J A Kemp
(86) International application number: PCT/US2008/055856
(87) International publication number: WO 2008/124229

(56) References cited:
- EP-A- 0 475 303
- WO-A-91/07485
- WO-A1-2006/137778
- US-A- 5 512 474
- US-A- 5 912 177
- US-A- 5 948 655
- US-A- 6 057 150
- US-A1- 2005 032 205
- US-B1- 6 372 495
- US-B1- 6 465 252

## Description

Stem cells are a category of undifferentiated cells that demonstrate potential in various therapeutic applications, including organ transplantation, tissue regeneration, blood transfusion, and bone marrow transplantation. To grow stem cells in amounts useful for therapeutic applications, an efficient and reliable mechanism for expanding stem cells is important. To be economically useful, such a mechanism should ensure that large numbers of stem cells are produced, in a manner that minimizes the chances of contaminating the stem cells.

Mammalian cells require homeostasis to survive; therefore, when growing human cells ex vivo, certain environmental parameters, including temperature, oxygen concentration, pH, osmolarity, nutrient concentrations, and ion concentrations must be carefully regulated. Further, in the context of stem cell expansion, many frequently grown cells such as mesenchymal stem cells (MSC), are anchorage-dependent. This means that when expanding MSCs ex vivo, their viability and proliferative capacity may be diminished unless they become anchored to a fixed surface.

There thus arises a need for a system in which cells can be grown efficiently in immobilized culture while minimizing labor costs and contamination risks. The most rudimentary bioreactor system involves the use of a polystyrene tissue-culture flask, but this method is impractical for all but the smallest-scale applications: culturing cells in culture flasks is labor-intensive, and has a high risk of contamination, owing to the fact that frequent opening of a culture flask increases the probability of contamination in the flask. Many bioreactor systems developed in response to the problems associated with tissue-culture flasks are referred to as "closed" systems; in these systems, automated fluid flow delivers nutrients to the cells, therefore allowing fewer chances for contamination, affording improved control over the process, and better approximating the cells' physiological environment. Automated culture bioreactors may be of the flat-plate or the hollow-fiber variety; use of the hollow-fiber variety maximizes the surface area available for growing cells within a reactor of a given volume.

Consequently, effort has been devoted to developing bioreactor surfaces to which adherent cells will attach. The task of developing suitable surfaces for cell adhesion has been made more difficult by the fact that cells do not readily or tightly adhere to the materials from which present-day hollow fibers are made. A solution to these problems requires the development of a cell adhesion system capable of adhering cells to surfaces to which they do not naturally bind, and to keep the cells bound in spite of constant exposure to shear stresses caused by the flow of media over the cells.

The polymers used as the substrata in constructing cell growth surfaces in known membrane bioreactors include polystyrene, polypropylene, polyethylene, polymethylpentylene, saponified cellulose esters, polymethylacrylate, polycarbonate, polyesters, polyethersulfone, styrene-acrylonitrile, polyacrylonitrile, PVC, organosilicone, cellulose ester, and polyamide.

Some polymeric surfaces have been treated with cell adhesion factors such as laminin, collagen, and fibronectin, either via covalent attachment or electrostatic adsorption. Other surfaces are treated using cell adhesion factors to physically anchor cells to membrane surfaces as shown in U.S. Patent No. 5,912,177. However, the use of cell adhesion factors by themselves is recognized in the art as insufficient to mediate the long-term attachment of cells to a polymeric matrix, as described in U.S. Patents, Nos. 5,512,474; 5,912,177, and as described, for example, by Prichard, H., Reichert, W. et al. in "Adult Adipose-Derived Stem Cell Attachment to Biomaterials" Biomaterials 28(6) 936-946. Furthermore, fibronectin and other cell-adhesion factors are expensive, and the current adsorptive techniques for fibronectin coating of polymeric surfaces are highly wasteful.

It is therefore desirable to find ways of either using fibronectin more efficiently, or to find ways of obviating the need for fibronectin.

US 6,465,252 discloses a cell culture method.

The invention will be described with reference to the drawings, in which:
FIG. 1 is a schematic view of a bioreactor useful in this invention.
FIG. 2 is a flow diagram of a cell expansion system that may be used with the present invention.
FIG. 3 is a graph comparing the effects of various surface treatments on mesenchymal stem cell growth.
FIG. 4 is another graph comparing the effects of various surface treatments on mesenchymal stem cell growth.

The invention is defined in the claims.

This invention is directed towards a method for promoting adhesion of mammalian cells to a membrane surface in a bioreactor comprising: providing a bioreactor comprising: a housing; and a polymeric membrane comprising at least one surface inside the housing; and treating the at least one polymeric membrane surface with at least a first surface treatment in an amount sufficient to improve cell adhesion to the at least one polymeric membrane surface, the first surface treatment being allowed to incubate with the at least one polymeric membrane surface for an amount of time sufficient to allow adsorption of the first surface treatment in an amount sufficient to promote enhanced cell adhesion; wherein the step of treating the at least one polymeric membrane surface with said first surface treatment comprises treating the at least one membrane surface with platelet lysate.

As discussed in the background, there are multiple ways of configuring a bioreactor in order to grow adherent mammalian cells; this invention is not dependent on any one configuration thereof. As used herein, the term "adherent mammalian cells" refers to any type of eukaryotic cells possessing a mammalian nuclear genome and adherent potential, regardless of species of origin, tissue of origin, cell lineage, or length of time in culture.

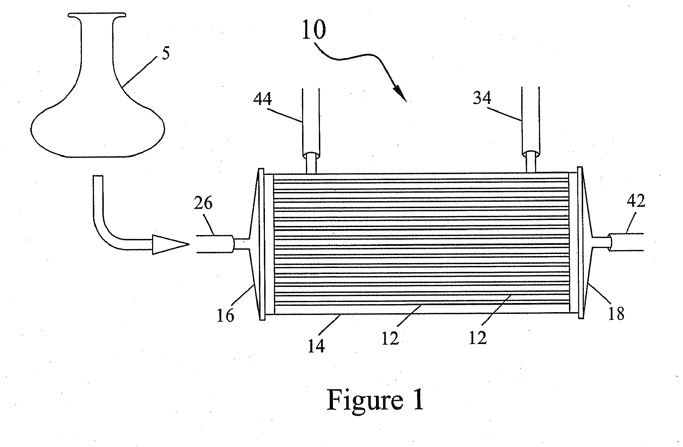

One non-limiting example of the embodiments of the present invention is the hollow-fiber bioreactor shown in FIG. 1. The bioreactor, or cell-expansion module **10** is made from a bundled set of biocompatible polymeric membranes **12** in the geometric form of hollow fibers, enclosed within a housing **14**. For purposes of this description, the set of all the hollow fibers, and both the intracapillary (IC) and extracapillary (EC) sides of them, is referred to as a membrane. The terms "membrane", "cell culture surface", "culture surface", "polymeric membrane", and "polymeric surface" are synonymous. The housing, or module **14** containing the fibers **12** may be cylindrical in shape, and may be made from any biocompatible polymeric material. The intracapillary side of the membrane is defined for purposes of this description as the luminal side of, and the volume enclosed by, or substantially enclosed by, a membrane resembling a hollow fiber. The extracapillary side of the membrane is defined for purposes of this description as any component of the volume within the bioreactor housing that is not enclosed by, or in contact with the luminal side of the hollow fibers. For purposes of this description, it is assumed that cells will be seeded, grown, and reseeded in the IC space only; this assumption is not intended to limit the scope of the claims, and it is understood that in the alternative, cells could be grown in the EC space, and the same principles described here would apply.

Each end of the module, or housing, is closed off with end caps, or headers **16**, **18**. These end caps **16**, **18** may be made of any suitable material such as polycarbonate so long as the material is biocompatible with the types of cells to be grown in the bioreactor.

The module has at least one port for entry and exit of fluids into the module; the module of an embodiment, as a nonlimiting example, has four ports. Two of the four ports fluidly connect to the extracapillary space. One port **34** is used for fluid and solute ingress into the extracapillary space, and the other port **44** is used for fluid and solute egress from the extracapillary space. The other two of the four ports fluidly connect to the intracapillary space; as a nonlimiting example, one port **26** is used for fluid and solute ingress into the intracapillary space, and the other port **42** is used for fluid and solute egress from the intracapillary space. It is by means of the aforesaid inlet ports **34**, **26** that cells, treatments, and media may be introduced into the bioreactor, and it is by means of the egress ports **44**, **42** that cells, treatments, and media may be removed from the bioreactor at times. For purposes of the invention, any physical aperture in a bioreactor **10** that allows ingress of material into the bioreactor is an inlet port; any port through which egress of material from the bioreactor occurs is an egress port.

The IC space is assumed to serve as a cell-growth chamber; however, as stated before, this assumption is nonessential to the invention, as cells may also be flowed into, and grown in the EC space. At the start of a new cell expansion period, cells may be flowed into the IC space. The IC space may be loaded with cells using a syringe, or from a bag containing a preparation of cells. The cells may be flowed into the IC space in cell culture media, or directly as bone marrow aspirate.

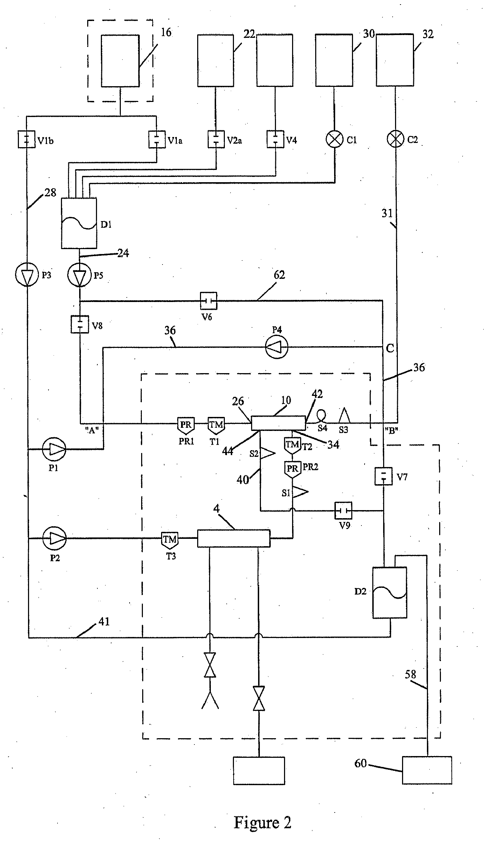

In an embodiment, an IC media bag **22** (see FIG. 2) may be connected via a portion of flexible tubing (the IC inlet line) **24** to the IC inlet port **26** of the bioreactor **10**. The IC inlet line **24** brings fresh IC media to the IC side of the bioreactor. Additional tubing line **62** can be added to the system as needed to enable specific applications such as reseeding or redistributing cells in the bioreactor.

A cell input bag **30** contains the cells to be expanded in the bioreactor **10**. The cell input bag **30** is connected to the IC inlet line **24** that delivers cells into the lumen of the hollow fibers via IC inlet port **26**.

When the cells are ready to be harvested, they are flushed out of the IC outlet port **42** of bioreactor **10** through cell harvest line **31** and into a cell harvest bag **32**.

The cell growth system also may include a length of tubing which acts as an IC re-circulation loop **36**. The IC media flows out of the bioreactor **10** from the IC outlet port **42** through tubing loop **36** and back into the bioreactor through the IC inlet port **26**. This loop **36** is used to recirculate the IC media though the hollow fibers. It may also be used to flush the cells out of the hollow fibers and reseed/redistribute them throughout the hollow fibers for further expansion as more fully described below.

As seen in FIG. 1, the space between the fibers **12** themselves, or EC space, may serve as a nutrient reservoir and a waste-collection site for the cells in the intracapillary space. Nutrients enter the IC space from the EC space by means of diffusion across the polymeric membrane; further, cellular waste products leave the IC space via the EC space. The EC media may be replaced at intervals to remove cell metabolic wastes, or may be continuously replaced. The EC media may be circulated as needed through an oxygenator **(4,** see FIG. 2). The EC media may be introduced into the bioreactor from an EC media bag (**16**, see FIG. 2), which in an embodiment is fluidly connected via a length of flexible tubing, or conduit **28** to EC inlet port **34**. The EC media, along with any cellular wastes, may be flushed from the bioreactor via EC egress port **44**, which is fluidly connected through a length of flexible tubing, or conduit **58**, to a waste bag **60**.

Also an EC recirculation loop including lines **40** and **41** may be provided to recirculate EC media. Again, if cells were being grown in the EC space, the IC media would serve as a nutrient reservoir and a waste collection pool for the cells.

A second assumption made solely for purposes of this description is that the fluid flowing through the IC space and the fluid flowing through the EC space flow opposite directions. This assumption is nonessential to the invention, as the invention may be used in a bioreactor in which fluid flows the same direction in both the EC and IC spaces.

The hollow fibers **12** in the particular embodiments here described are approximately 9000 in number, and are approximately 295 mm in length. They may be held in place within the housing by polyurethane potting (not shown). The fibers **12** and the potting may be cut through cross-sectionally, to permit fluid flow through the IC space. It is understood that the length and number of the fibers **12** may be varied; the embodiments here described are merely exemplary.

The hollow fibers **12** may be made of a semi-permeable, biocompatible, polymeric material. One such polymeric material is a blend of polyamide and polyarylethersulfone. The semi-permeable membrane allows transfer of nutrients, wastes, and gases through the membrane between the EC and IC spaces. Exchange of fluid takes place in part because the fibers have a generally porous consistency, which facilitates diffusion and convection of molecules across the membranes.

One embodiment of the membrane **12** comprises 65-95% by weight of at least one hydrophobic polymer and 5-35% by weight of at least one hydrophilic polymer. The hydrophobic polymer may be chosen from the group consisting of polyamide (PA), polyaramide (PAA), polyarylethersulfone (PAES), polyethersulfone (PES), polysulfone (PSU), polyarylsulfone (PASU), polycarbonate (PC), polyether (PE), polyurethane (PUR), polyetherimide, and copolymer mixtures of any of the above polymers, such as polyethersulfone, or a mix of polyethersulfone and polyamide. The hydrophilic polymer may be chosen from the group consisting of polyvinylpyrrolidone (PVP), polyethylene glycol, (PEG), polyglycolmonoester, water- soluble cellulosic derivatives, polysorbate, and polyethylene-polypropylene oxide copolymers.

The polymeric hollow fibers **12** may be treated with a substance, or "surface treatment" to improve the adherence of the cells to the membrane, especially if adherent cells, or anchorage-dependent cells are to be grown in the bioreactor. The terms "treat", "treated", or "treating" mean that substantially all portions of the cell culture surfaces of the hollow fibers have been subjected to a surface treatment for an amount of time sufficient to allow the treatment molecules to become adsorbed to the membrane. Further, covalent, adsorbed, and soluble treatments may be used in conjunction with one another, without restriction as to combinations or amounts.

### Methods

The steps of treating the membrane or cell culture surface with a surface preparation may be conducted as follows: prior to the membrane treating step, the cell culture surface **12** is primed by wetting with a saline solution, which in an embodiment is PBS, or phosphate buffered saline. To avoid the formation of precipitates, the PBS must be free of divalent cations such as Mg⁺⁺ or Ca⁺⁺.

Following the priming procedure, the membrane is treated with a surface treatment of platelet lysate (PL).

For purposes of this invention, human platelet lysate is a solution containing plasma and lysed human platelets. The solution may be prepared by any method of causing human platelets to lyse, including those methods currently known in the art. In one method, 1.5 x 10⁹ / mL platelets in plasma is frozen at -80 °C to lyse the platelets; the resulting biologically active solution is hereafter known as platelet lysate solution. This platelet lysate is thawed and centrifuged at 1000 x g for 10 minutes. The resulting supernatant is used to treat the membrane.

The surface treatment is allowed to contact the membrane **12** by pumping or dripping the surface treatment into the IC space.

The surface treatment may be introduced into the bioreactor by itself, or may be included in the cell culture media. In an alternate embodiment, one surface treatment may be introduced into the bioreactor along with another surface treatment which is different from the first surface treatment.

Once in contact with the membrane of the bioreactor, the surface treatment is allowed to incubate with the membrane for an amount of time sufficient to allow adsorption of the surface treatment in an amount sufficient to promote enhanced cell adhesion.

Cell loading may be accomplished by sending aqueously-suspended cell samples into the bioreactor **10** via the IC inlet port **26**. As discussed above, platelet lysate or plasma may also be included with the cells to be expanded.

### EXAMPLES

### Example 1

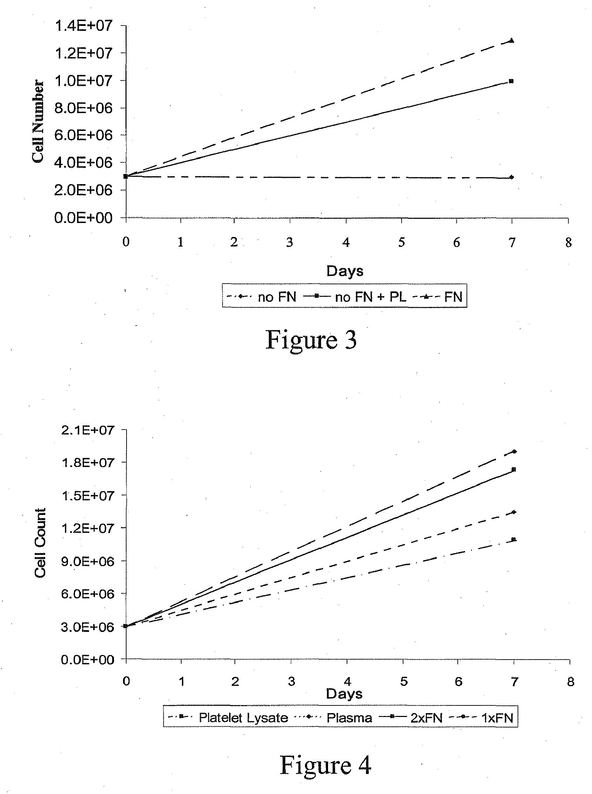

Three polyflux hollow fiber bioreactors were used in this example. One bioreactor-was not treated with anything (referred to in FIG. 3 as no FN). One bioreactor was treated with fibronectin (FN) and one was treated with platelet lysate (no FN + PL) according to the above-described methods. Around 3 x 10⁶ mesenchymal stem cells were loaded into each bioreactor on day 0. The cells were grown for seven days. The EC and IC media was replaced on days three and five and the cells were harvested and counted on day seven.

As can be seen from FIG. 3, the bioreactors treated with either fibronectin (FN) or platelet lysate (PL) produced much better cell expansion than the untreated bioreactor. Increased cell numbers produced by the bioreactors with the treated fibers indicate that cells were able to attach to the membrane and grow.

### Example 2

Four polyflux hollow fiber bioreactors were used in this example. One bioreactor was treated with an amount of fibronectin (1x FN), one bioreactor was treated with twice the amount of fibronectin (2x FN), one bioreactor was treated with platelet lysate and one was treated with plasma according to the above-described methods. Around 3 x 10⁶ mesenchymal stem cells were loaded into each bioreactor on day 0. The cells were grown for seven days. The EC and IC media was replaced on days three and five and the cells were harvested and counted on day seven.

As can be seen from FIG. 4, the bioreactors treated with either 1x or 2x fibronectin produced the highest cell expansion. However, cells grown on membranes treated with platelet lysate and plasma also showed good expansion in culture.

The examples given above are several of the applications which could be utilized following the principals of the present invention and are not meant to limit the scope of the present invention as defined by the attached claims.

## Claims

1. A method for promoting adhesion of mammalian cells to a membrane surface in a bioreactor (10) comprising:
providing a bioreactor (10) comprising:
a housing (14); and
a polymeric membrane (12) comprising at least one surface inside the housing (14); and
treating the at least one polymeric membrane surface with at least a first surface treatment in an amount sufficient to improve cell adhesion to the at least one polymeric membrane surface, the first surface treatment being allowed to incubate with the at least one polymeric membrane surface for an amount of time sufficient to allow adsorption of the first surface treatment in an amount sufficient to promote enhanced cell adhesion;
wherein the step of treating the at least one polymeric membrane surface with said first surface treatment comprises treating the at least one membrane surface with platelet lysate.

2. The method of claim 1, wherein the polymeric membrane (12) comprises at least one hydrophobic polymer and at least one hydrophilic polymer.

3. The method of claim 1 or 2, wherein the polymeric membrane is a hollow fiber membrane.

4. The method of claim 1, 2 or 3, further comprising treating the at least one polymeric membrane surface with a second surface treatment which is different from the first surface treatment.

5. The method of any one of the preceding claims, wherein said polymeric membrane is a hollow-fiber semi-permeable polymeric membrane (12) having an intracapillary space surface and an extracapillary space surface.

6. The method of claim 5, wherein said at least one polymeric membrane surface is said intracapillary space surface.

## Patentansprüche

1. Verfahren zum Fördern einer Haftung von Säugetierzellen an einer Membranoberfläche in einem Bioreaktor (10), umfassend:
Bereitstellen eines Bioreaktors (10), umfassend:
ein Gehäuse (14); und
eine Polymermembran (12), umfassend mindestens eine Oberfläche in dem Gehäuse (14); und
Behandeln der mindestens einen Polymermembranoberfläche mit mindestens einer Oberflächenbehandlung in einer Menge, die ausreichend ist, um eine Zellhaftung an der mindestens einen Polymermembranoberfläche zu verbessern, wobei zugelassen wird, dass die erste Oberflächenbehandlung mit der mindestens einen Polymermembranoberfläche für eine Zeitdauer inkubiert, die ausreichend ist, um eine Adsorption der ersten Oberflächenbehandlung in einer Menge zuzulassen, die ausreichend ist, um eine verbesserte Zellhaftung zu fördern;
wobei der Schritt des Behandelns der mindestens einen Polymermembranoberfläche mit der ersten Oberflächenbehandlung ein Behandeln der mindestens einen Membranoberfläche mit Blutplättchenlysat umfasst.

2. Verfahren nach Anspruch 1, wobei die Polymermembran (12) mindestens ein hydrophobes Polymer und mindestens ein hydrophiles Polymer umfasst.

3. Verfahren nach Anspruch 1 oder 2, wobei die Polymermembran eine Hohlfasermembran ist.

4. Verfahren nach Anspruch 1, 2 oder 3, ferner umfassend ein Behandeln der mindestens einen Polymermembranoberfläche mit einer zweiten Oberflächenbehandlung, die anders als die erste Oberflächenbehandlung ist.

5. Verfahren nach einem der vorhergehenden Ansprüche, wobei die Polymermembran eine semipermeable Hohlfaserpolymermembran (12) ist, die eine intrakapilläre Raumoberfläche und eine extrakapilläre Raumoberfläche hat.

6. Verfahren nach Anspruch 5, wobei die mindestens eine Polymermembranoberfläche die intrakapilläre Raumoberfläche ist.

## Revendications

1. Procédé destiné à favoriser l'adhésion de cellules de mammifère à une surface membranaire dans un bioréacteur (10) comprenant :
la fourniture d'un bioréacteur (10) comprenant :
un boîtier (14) ; et
une membrane polymère (12) comprenant au moins une surface à l'intérieur du boîtier (14) ; et
le traitement de l'au moins une surface membranaire polymère avec au moins un premier traitement de surface dans une quantité suffisante pour améliorer l'adhésion cellulaire à l'au moins une surface membranaire cellulaire, le premier traitement de surface pouvant incuber avec l'au moins une surface membranaire polymère pendant une quantité de temps suffisante pour permettre l'adsorption du premier traitement de surface dans une quantité suffisante pour favoriser l'adhésion cellulaire améliorée,
l'étape de traitement de l'au moins une surface membranaire polymère avec ledit traitement de surface comprenant le traitement de l'au moins une surface membranaire avec un lysat de plaquettes.

2. Procédé selon la revendication 1, dans lequel la membrane polymère (12) comprend au moins un polymère hydrophobe et au moins un polymère hydrophile.

3. Procédé selon la revendication 1 ou 2, dans lequel la membrane polymère est une membrane fibreuse creuse.

4. Procédé selon la revendication 1, 2 ou 3, comprenant en outre le traitement de l'au moins une surface membranaire polymère avec un deuxième traitement de surface qui est différent du premier traitement de surface.

5. Procédé selon l'une quelconque des revendications précédentes, dans lequel ladite membrane polymère est une membrane polymère semi-perméable fibreuse creuse (12) présentant une surface d'espace intracapillaire et une surface d'espace extracapillaire.

6. Procédé selon la revendication 5, dans lequel ladite au moins une surface membranaire polymère est ladite surface d'espace intracapillaire.
